# EUROPÄISCHE PATENTSCHRIFT

(11) **EP 0 713 689 B1**
(45) Veröffentlichungstag und Bekanntmachung des Hinweises auf die Patenterteilung: **04.07.2001**
(21) Anmeldenummer: 95105256.2
(22) Anmeldetag: 07.04.1995
(51) Int. Cl.: A61F 2/64

(54) **Schwenkvorrichtung zwischen Teilen eines orthopädischen Hilfsmittels**
Pivoting device between parts of an orthopedic aid
Dispositif de pivotement entre parts d'un moyen orthopédique

(30) Priorität: 25.11.1994 NL 9401975
(43) Veröffentlichungstag der Anmeldung: 29.05.1996
(73) Patentinhaber: Otto Bock Orthopädische Industrie Besitz- und Verwaltungs-Kommanditgesellschaft, 37115 Duderstadt (DE)
(72) Erfinder: Van de Veen, P.G. Dr. Ing., NL 7514 EC Enschede (NL)
(74) Vertreter: Gramm, Werner, Prof. Dipl.-Ing.

(56) Entgegenhaltungen:
- EP-A- 0 439 028
- EP-A- 0 672 398
- CH-A- 274 375
- DE-A- 1 491 233
- DE-C- 735 844

## Beschreibung

Die Erfindung betrifft eine Schwenkvorrichtung zwischen Teilen eines orthopädischen Hilfsmittels, wie z.B. ein Prothesenknie für Beinamputierte, bestehend aus einer mehrgliedrigen kinematischen Gelenkkette mit mindestens vier Gelenkgliedern, in der die jeweils miteinander verbundenen Gelenkglieder eine gemeinsame Drehachse aufweisen, und diese Drehachsen im wesentlichen zueinander parallel verlaufen.

Eine derartige Vorrichtung ist in der älteren aber nicht vorveröffentlichten europäischen Patentanmeldung 95102056.9, veröffentlicht mit der Nummer EP-A-0 672 398, beschrieben. Bei dieser Schwenkverbindung ist ein streckseitig angeordnetes Gelenkglied nur mit einem oberen Anschluß-Gelenkglied und einem unteren Anschluß-Gelenkglied drehbar verbunden. Ein beugeseitig angeordnetes Gelenkglied ist mit seinem einen Ende mit dem oberen Anschluß-Gelenkglied drehbar und mit seinem anderen Ende mit dem unteren Anschluß-Gelenkglied dergestalt verbunden, daß bei Bewegung der Schwenkverbindung das beugeseitig angeordnete Gelenkglied gegenüber dem unteren Anschluß-Gelenkglied eine zwangsläufige Bewegung ausführt, die sowohl Translation als auch Rotation umfaßt. Diese Schwenkverbindung ermöglicht eine relativ große Standphasen-Beugung bei gleichzeitiger Verhinderung eines zweiten Freiheitsgrades. Dabei ist die Standphasen-Beugung groß genug, um ein natürliches Gehen zu ermöglichen, in der die eine Schwenkverbindung (Standphasen-Beugung) die andere Schwenkverbindung (Schwungphasen-Beugung) blockiert. Dabei gibt die Schwenkverbindung die Möglichkeit, den Anschlußpunkt für ein orthopädisches Hilfsmittel ausreichend weit zur Streckseite zu legen, und zwar auch im Fall einer Knie-Exartikulation.

Wichtig bei einer derartigen, durch eine mehrgliedrige kinematische Gelenkkette gebildeten Schwenkverbindung ist bei Verwendung als Kniegelenk, daß die Schwenkverbindung unter Belastung eine initiale einfedernde Beugung aufweist entsprechend dem menschlichen Kniegelenk unter Belastung. Der dämpfende Einfluß dieser initialen einfedernden Beugung des Gelenkes bei Belastung des Beins verhindert eine ruckende Gangart, die für den Benutzer unangenehm und auf Dauer auch schmerzhaft sein kann. Die initiale Beugung des Gelenkes verhindert ferner eine Beschränkung der senkrechten Bewegung des Körperschwerpunktes, wodurch die für das Gehen erforderliche Energie beschränkt bleibt. Diese Kniebeugung wird "Stance Flexion" genannt.

Die bekannten Schwenkvorrichtungen zwischen Teilen eines orthopädischen Hilfsmittels, wie beispielsweise einer Prothese für Beinamputierte, weisen zumindest einen der nachfolgenden Nachteile auf:
- Sie weisen die beschriebenen Eigenschaften überhaupt nicht auf.
- Sie weisen die beschriebenen Eigenschaften nur in geringem Maße auf. Dies ist der Fall, wenn die in der Praxis auftretende "Stance Flexion" nicht größer als 3° bis 4° ist, was zu gering ist, als daß die beschriebenen Vorteile der Stance Flexion vollständig realisiert werden können.
- Sie realisieren die beschriebenen Eigenschaften durch Verwendung eines mehrgliedrigen Gelenksystems mit zwei Freiheitsgraden, die beide als die besagte Schwenkbewegung des Mechanismus verstanden werden können, wobei die Bewegung nach einem einzigen Freiheitsgrad gegen die Federkraft eines elastischen Elements erfolgt und wobei die beiden Freiheitsgrade sich gegenseitig wohl beeinflussen, jedoch trotzdem unabhängig voneinander sein können. Diese Mechnismen haben den Nachteil, daß der Austausch gespeicherter Federenergie zwischen diesen Freiheitsgraden immer möglich ist, was vom Benutzer als äußerst unangenehmer Effekt erfahren wird, so daß zusätzliche Mittel, beispielsweise dissipative Elemente, erforderlich sind, um diese Federenergie durch Dissipation in Wärme umzuwandeln.
- Sie realisieren die beschriebenen Eigenschaften durch Verwendung eines mehrgliedrigen Gelenksystems mit nur einem einzigen Freiheitsgrad und einem Wendepunkt in der Schwenkbewegung, was dadurch erreicht wird, daß in der Verlängerung eines der Glieder des mehrgliedrigen Gelenksystems ein zweites Glied oder eine Schubverbindung angebracht wird, was solche Mechanismen relativ lang macht.

Aufgabe der Erfindung ist es, eine Vorrichtung zu schaffen, mit der eine "Stance Flexion" realisiert werden kann, die so groß ist, daß die Vorteile der Stance Flexion, wie sie das natürliche Kniegelenk aufweist, in vollem Umfange realisiert werden.

Weiter hat die Erfindung die Aufgabe, diese beschriebene Aufgabe ohne die vorstehend genannten Nachteile bestehender Vorrichtungen zu lösen. Eine weitere Aufgabe der Erfindung ist die Schaffung einer Vorrichtung, die eine einfache Konstruktion mit großer Zuverlässigkeit kombiniert.

Ausgehend von der eingangs beschriebenen Schwenkvorrichtung werden die vorstehend genannten Aufgaben erfindungsgemäß dadurch gelöst, daß in der Ausgangsposition des mehrgliedrigen Gelenksystems ein Gelenkglied gegenüber einem mit ihm verbundenen Gelenkglied zwei unterschiedliche Bewegungen ausführen kann, wobei jede dieser beiden Bewegungen eine Rotation, eine Translation oder eine kombinierte Rotation/Translation sein kann, und wobei die Einleitung einer dieser Bewegungen die andere der beiden möglichen Bewegungen zumindest über ihren größten Bewegungsbereich blockiert.

Unter dem verwendeten Begriff "Bewegung" sind somit eine reine Drehung bzw. Rotation, eine reine Translation oder aber jede feste Kombination einer Rotation mit einer Translation zu verstehen. Der Begriff "Bewegung" beschreibt somit die Bewegung nach einem einzigen typischen Bewegungs-Freiheitsgrad der Schwenkvorrichtung.

Die erfindungsgemäße Schwenkvorrichtung führt zu der gewünschten Flexibilität und ermöglicht eine relativ große "Stance Flexion" in Verbindung mit einer weitgehenden Blockierung des jeweils zweiten Bewegungs-Freiheitsgrades. Diese Ergebnisse werden mit einem relativ kleinen und einfach zu realisierenden Mechanismus erreicht.

Die Unteransprüche 2 bis 11 beschreiben vorteilhafte Ausführungsformen der Erfindung, wobei das zusätzliche federnde Element gemäß Anspruch 10 beispielsweise dazu verwendet werden kann, den Unterschenkel zum Ende der Schwungphase leicht nach vorne zu bringen.

Weitere Merkmale der Erfindung werden anhand der in den nachfolgenden Figuren dargestellten Ausführungsbeispiele der Erfindung erläutert, die hierauf allerdings nicht beschränkt ist. Es zeigen:
- Figur 1: eine Seitenansicht eines erfindungsgemäßen Prothesenkniegelenkes, angeordnet zwischen einem künstlichen Oberschenkel und einem künstlichen Unterschenkel;
- Figur 2: das Prothesenkniegelenk gemäß Figur 1 in vergrößertem Maßstab in teilweise geschnittener perspektivischer Ansicht;
- Figur 3: eine schematische Darstellung in der Seitenansicht des Prothesenkniegelenkes gemäß Figur 2 im Ruhezustand;
- Figur 4: eine mit der Figur 3 übereinstimmende Ansicht des Prothesenkniegelenkes unter Gewichtsbelastung;
- Figur 5: eine teilweise geschnittene perspektivische Ansicht einer Variante in belastetem Zustande;
- Figur 6: eine mit der Figur 4 übereinstimmende Ansicht des Prothesenkniegelenkes gemäß Figur 5 in belastetem Zustand;
- Figur 7: eine mit der Figur 6 übereinstimmende Ansicht des Prothesenkniegelenkes in unbelastetem Zustand;
- Figur 8: eine weitere geänderte Ausführung in teilweise geschnittener perspektivischer Ansicht im Zustand ohne Gewichtsbelastung;
- Figur 9: eine mit den Figuren 3 und 7 übereinstimmende Ansicht des Prothesenkniegelenkes gemäß Figur 8 im Ruhezustand;
- Figur 10: eine mit der Figur 9 übereinstimmende Ansicht des Prothesenkniegelenkes gemäß Figur 8 unter Gewichtsbelastung;
- Figur 11: eine mit der Figur 6 übereinstimmende Ansicht einer Variante; und
- die Figuren 14, 15, 16; 17, 18, 19; 20, 21, 22; 23, 24, 25; 26, 27, 28: in der schematischen Seitenansicht fünf weitere Ausführungsbeispiele, jeweils in drei Zuständen.

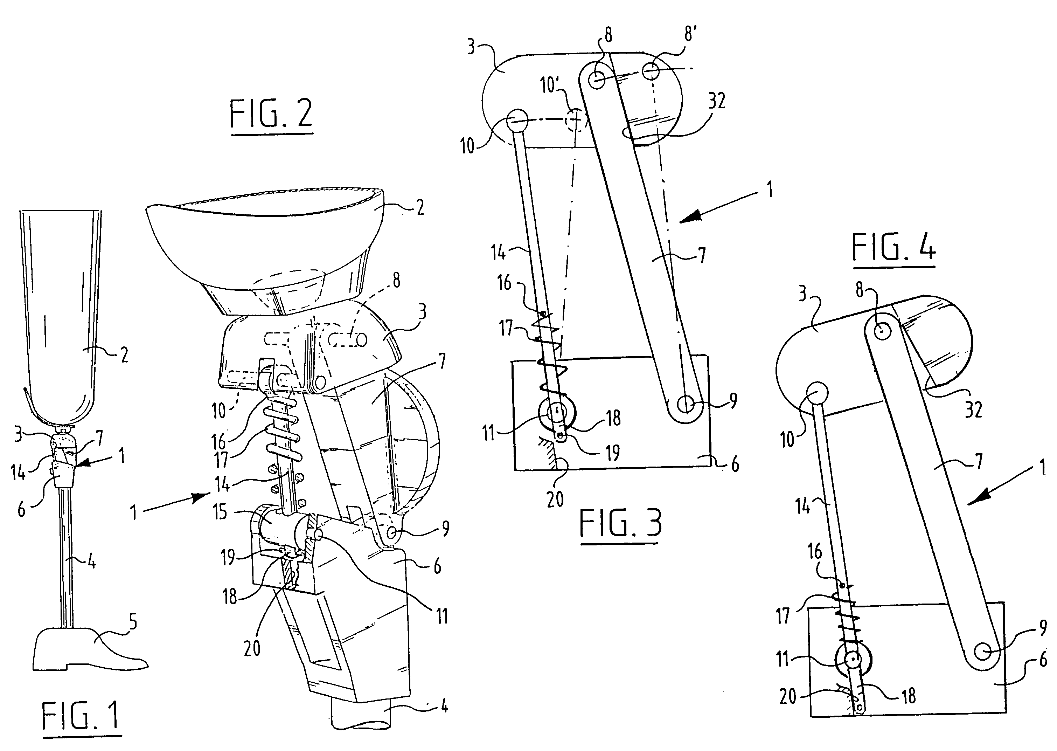

Figur 1 zeigt ein Prothesenkniegelenk 1, das einen mit einem künstlichen Oberschenkel 2 verbundenen ersten Teil 3 und einen mit einem künstlichen Unterschenkel 4 mit Fuß 5 verbundenen unteren Teil 6 umfaßt. Weiter umfaßt das Prothesenkniegelenk 1 eine nachstehend beschriebene kinematische Gelenkkette, zu der die Teile 3 und 6 gehören.

Die Drehachsen (Drehgelenke) 8, 9, 10, 11 sind alle untereinander im wesentlichen parallel und bestimmen eine Bewegungsebene, die senkrecht zu ihnen verläuft.

Mit der Drehachse 11 ist ein Führungsblock 15 gelenkig verbunden, in dem das hintere Gelenkglied 14 schiebbar geführt ist. Zwischen dem Block 15 und einem am Gelenkglied 14 vorhandenen Bund 16 ist eine Druckfeder 17 angebracht. Diese Druckfeder drückt das Gelenkglied 14 in der Weise nach oben, daß dadurch eine Ruhelage des oberen Teils 3 gegenüber dem unteren Teil 6 bestimmt wird. In dieser in Figur 3 schematisch dargestellten unbelasteten Ruhelage ist der obere Teil 3 frei schwenkbar gegenüber dem unteren Teil 6. Als Beispiel sind mit unterbrochenen Linien rotierte Positionen der Drehachsen (Drehgelenke) 10 und 8 angegeben und mit 10' beziehungsweise 8' gekennzeichnet.

Figur 4 zeigt die Situation, in der unter Gewichtsbelastung das hintere Gelenkglied 14 entgegen der Wirkung der Druckfeder 17 nach unten versetzt worden ist. In dieser Stellung wirkt der Endbereich 18 mit einem Anschlag 20 in Form einer Anschlagsfläche an dem unteren Teil 6 zusammen. Dadurch ist der in der Figur 3 mit unterbrochenen Linien dargestellte Rotations-Freiheitsgrad gesperrt.

Es wird angemerkt, daß der Endbereich 18 einen Querstift 19 trägt, der verhindert, daß das Gelenkglied 14 den Block 15 unter der Einwirkung der Feder 17 verläßt.

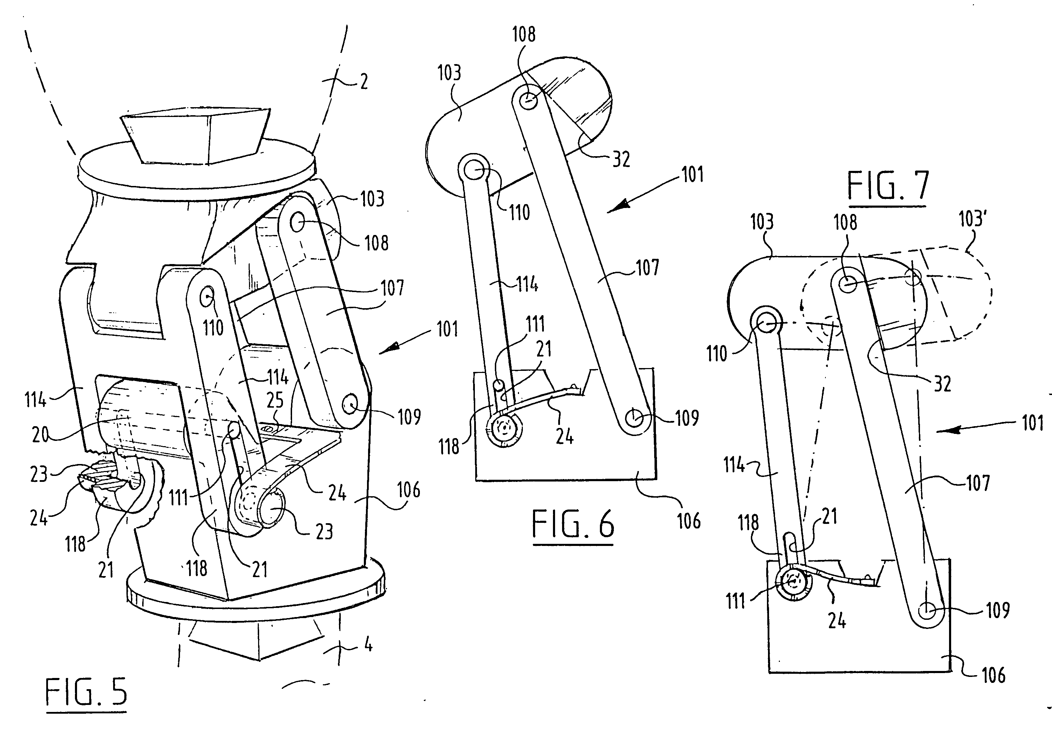

Die Figuren 5, 6 und 7 zeigen eine Variante. Das Prothesenkniegelenk 101 umfaßt einen oberen Teil 103, einen unteren Teil 106, zwei vordere Gelenkglieder 107 und zwei miteinander gekoppelte hintere Gelenkglieder 114. Die jeweiligen Gelenke sind mit 108, 109, 110 beziehungsweise 111 bezeichnet. Anders als bei dem Prothesenkniegelenk 1 trägt der untere Teil 106 einen Anschlag 20 in Form eines Querstiftes, der verschiebbar in einem Langloch 21 im hinteren Gelenkglied 114 angeordnet ist. Funktional stimmt diese Anordnung mit der Führbarkeit des hinteren Gelenkgliedes 14 im Block 15 in Figuren 1-4 überein.

Der Endbereich 118 trägt einen Zapfen 23, auf dem das augenförmige Ende einer Blattfeder gelagert ist. Die Blattfeder ist mit einem Befestigungsteil 25 mit dem unteren Teil 106 verbunden. Sie wirkt einerseits als Rückstellfeder, um das Prothesenkniegelenk 101 in die in Figur 7 gezeigte Ruhelage zu bewegen, und andererseits als Führungsarm, durch den der Zapfen 23 und damit der Endbereich 118 in eine vorbestimmte Bahn gezwungen wird; und zwar in der Weise, daß der in der Figur 7 mit Strichlinien bezeichnete Rotations-Freiheitsgrad in der in den Figuren 5 und 6 gezeigten Stellung gesperrt ist. Die in den Figuren 5 und 6 dargestellte Stellung wird unter Gewichtsbelastung erreicht, insbesondere wenn ein Nutzer auf der mit dem Prothesenkniegelenk versehenen Beinprothese steht.

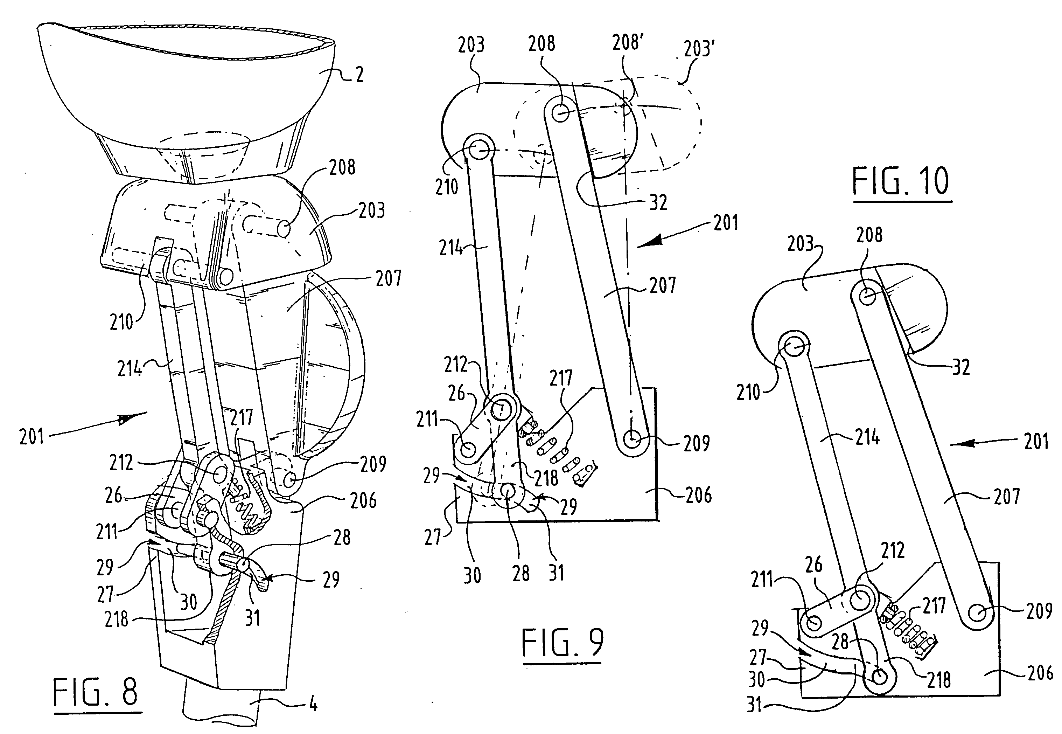

Die Figuren 8, 9 und 10 zeigen ein Prothesenkniegelenk 201. Dieses umfaßt einen oberen Teil 203 und einen unteren Teil 206, die durch ein vorderes Gelenkglied 207 und ein hinteres Gelenkglied 214 miteinander verbunden sind. Die jeweiligen Drehachsen (Drehgelenke), die den Achsen (Gelenken) 8, 9, 10, 11 in den Figuren 1 - 4 und den Achsen (Gelenken) 108, 109, 110, 111 in den Figuren 5-7 entsprechen, sind mit 208, 209, 210 beziehungsweise 211 bezeichnet.

Beim Prothesenkniegelenk 201 ist das hintere Gelenkglied 214 mittels einer fünften Drehachse 212 gelenkig mit einem Führungsarm 26 verbunden, der mittels der vierten Drechachse 211 mit dem hinteren Bereich 27 des unteren Teils 206 verbunden ist. Der freie Endbereich 218 des hinteren Gelenkgliedes 214 trägt einen Querstift 28, der in Langlöchern 29 im unteren Teil 206 geführt wird.

Wie bei den vorstehend beschriebenen Ausführungsbeispielen wird durch diese Gelenkgliedanordnung mit Zusatzführung erreicht, daß in dem in den Figuren 8 und 9 gezeigten freien Zustand, der durch eine Druckfeder 217 erreicht wird, eine freie Rotation um die Drehachsen 208, 209, 210, 212 gewährleistet wird. Mit unterbrochenen Linien ist ein rotierter Zustand des oberen Teils 203 gezeigt. Bei Gewichtsbelastung bewegt sich der obere Teil 103 nach unten und drückt dabei die Druckfeder 217 zusammen. Dadurch wird der Querstift 28 in den rechten Teil des Langlochs 29 bewegt. In diesem Zusammenhang wird angemerkt, daß der linke Teil 30 eine Krümmung aufweist, deren Krümmungsmittelpunkt die Drehachse 212 bildet. Dieser linke Teil 30 schließt fließend an den rechten Teil 31 an.

Alle drei Ausführungsbeispiele des erfindungsgemäßen Prothesenkniegelenkes haben einen Streckanschlag 32 gemeinsam, der an dem oberen Teil 3, 103 beziehungsweise 203 angebracht ist und mit dem oberen Teil des vorderen Gelenkgliedes 7, 107, 207 zusammenwirkt.

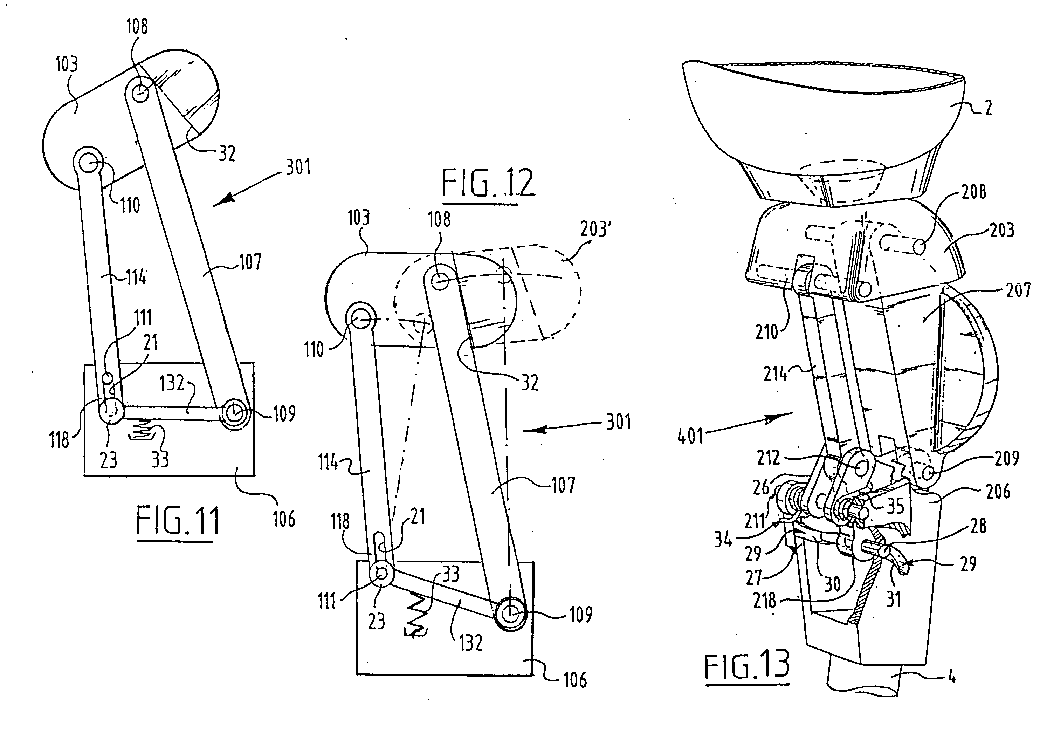

Die Figuren 11 und 12 zeigen ein Prothesenkniegelenk 301 in zwei Stellungen, entsprechend den Figuren 6 und 7. In dieser Ausführung ist die Blattfeder 24 gemäß den Figuren 5, 6 und 7 durch einen Lenker 132 ersetzt, der zwischen der zweiten Drehachse und dem Zapfen 23 angebracht ist sowie durch eine zwischen dem Lenker 132 und dem unteren Teil 106 angebrachte Druckfeder 33.

Figur 13 zeigt eine mit der Figur 8 übereinstimmende Ansicht eines Prothesenkniegelenkes 401. Abweichend von der Ausführung gemäß der Figur 8 erfolgt die Rückstellung des Prothesenkniegelenkes 401 durch Torsionsfedern 34, 35, die wie die Druckfeder 217 in dem Prothesenkniegelenk 201 die Funktion haben, den Arm 26 von einer zusammengedrückten Stellung (siehe Figur 10) aus in die mehr nach oben gerichtete Stellung (siehe Figuren 8 und 9) zu bewegen. Die Torsionsfedern 34, 35 wirken zwischen dem unteren Teil 206 und dem Arm 26.

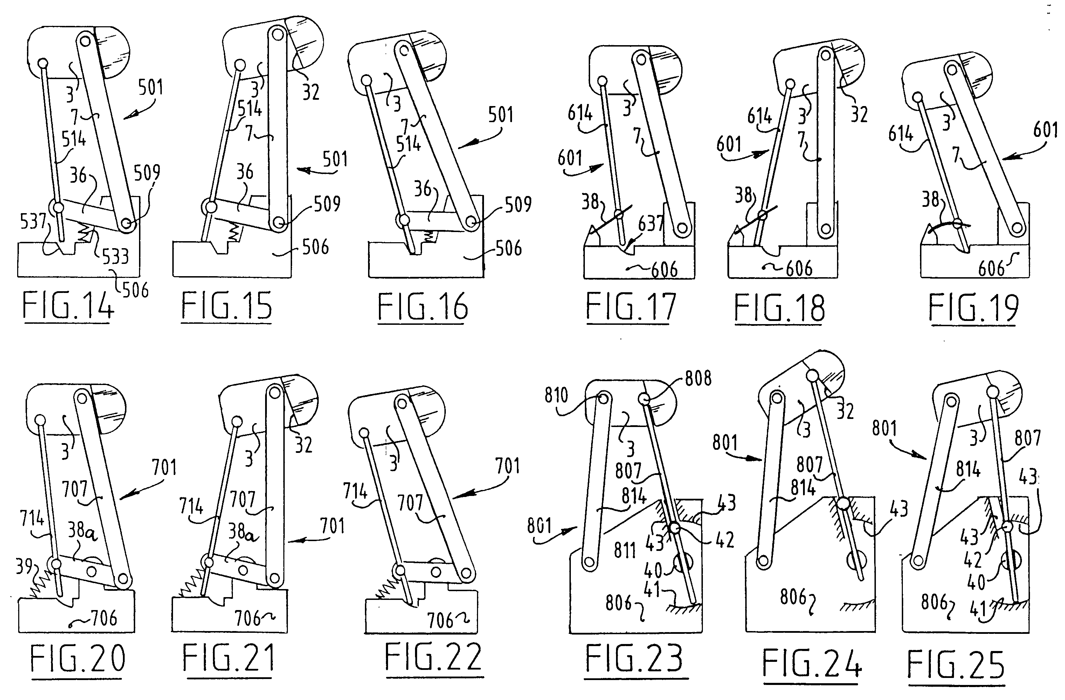

Die Figuren 14, 15 16; 17, 18, 19; 20, 21, 22; und 23, 24, 25 stellen vier verschiedene Prothesenkniegelenke 501, 601, 701 und 801 in drei unterschiedlichen Stellungen dar, nämlich in der Ausgangsstellung, in der Stellung nach der "Bewegung 1" oder der "Swing Flexion" und in der Stellung nach der "Bewegung 2" oder der "Stance Flexion".

Die einzelnen Stellungen bedürfen nach den vorstehenden detaillierten Beschreibungen der betreffenden Ausführungsbeispiele keiner näheren Erläuterung. Deshalb beschränken sich die nachfolgenden Ausführungen auf die Darlegung wesentlicher Unterschiede.

Das Prothesenkniegelenk 501 gemäß den Figuren 14, 15, 16 ist so ausgeführt, daß das hintere Gelenkglied 14 über den Lenker 36, mit dem es gelenkig verbunden ist, mit der Drehachse 509 gekoppelt ist. Auf den Lenker 36 wirkt die Feder 533. Das freie untere Ende des hinteren Gelenkgliedes 14 wirkt mit der Führungsfläche 537 zusammen.

Beim Prothesenknie 601 gemäß den Figuren 17, 18, 19 wird eine Blattfeder 38 verwendet, die mit dem unteren Teil 606 fest und mit dem hinteren Gelenkglied 14 an einer ortsfesten Position gelenkig verbunden ist. Das untere Ende des hinteren Gelenkgliedes 14 wirkt mit einer Führungsfläche 637 zusammen.

Das Prothesenkniegelenk gemäß den Figuren 20, 21, 22 weist die Besonderheit auf, daß das vordere Gelenkglied 707 nicht unmittelbar mit dem unteren Teil 706 gekoppelt ist. Stattdessen wird hier ein Kipparm 38a verwendet, der mit seinem mittleren Bereich mit dem unteren Teil 706, mit seinem vorderen Bereich mit dem vorderen Gelenkglied 707 und mit seinem hinteren Bereich mit dem hinteren Gelenkglied 714 jeweils gelenkig verbunden ist. Der Kipparm 38 wird von einer Druckfeder 39 in die in Figur 20 dargestellte Ruhelage oder Ausgangsstellung gebracht.

Das Prothesenkniegelenk 801 gemäß den Figuren 23, 24 und 25 weist die Besonderheit auf, daß das hintere Gelenkglied 814 über die Drehachsen 810 und 811 mit dem oberen Teil 3 bzw. mit dem unteren Teil 806 verbunden ist. Das vordere Gelenkglied 807 ist mittels Drehachse 808 mit dem oberen Teil 3 verbunden. Das Gelenkglied 807 wird im Schubgelenk 40 geführt, das mit dem unteren Teil 806 verbunden ist. Das untere Ende des Gelenkgliedes 807 wirkt mit der Führungsfläche 41 zusammen. Ein fest mit dem vorderen Gelenkglied 807 verbundenes Führungsorgan wirkt mit Führungsflächen zusammen, die alle mit 43 bezeichnet sind.

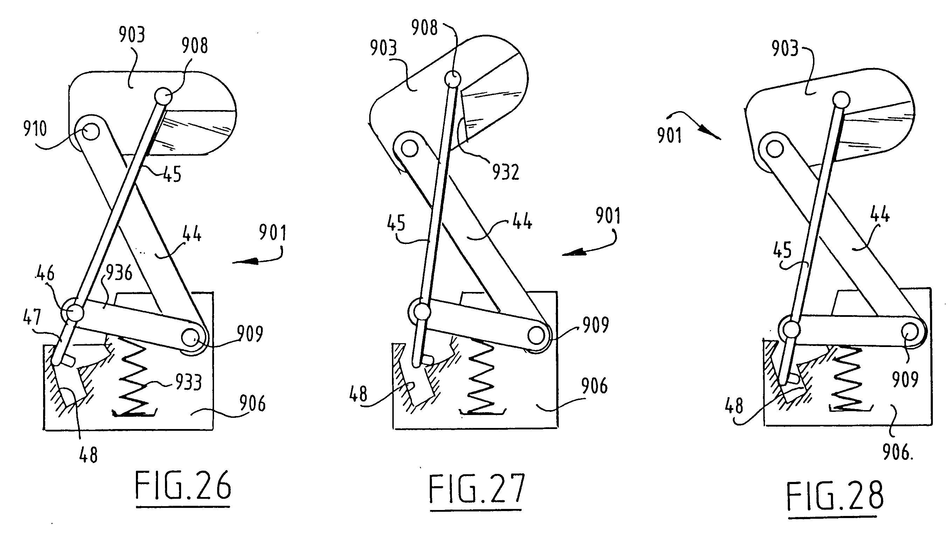

Das Prothesenkniegelenk 901 gemäß den Figuren 26, 27 und 28 umfaßt einen unteren Teil 906 und einen oberen Teil 903. Abweichend von allen vorstehend dargestellten und beschriebenen Ausführungsbeispielen umfaßt das Kniegelenk 901 zwei Gelenkglieder 44, 45, die, wie die Figuren deutlich zeigen, sich in dem Bereich zwischen den Teilen 903 und 906 kreuzen. Das Gelenkglied 44 ist mittels eines vorderen Gelenkes 909 mit dem unteren Teil 906 und mittels eines hinteren Gelenkes 910 mit dem oberen Teil 903 verbunden. Das Gelenkglied 45 ist mittels eines vorderen Gelenkes 908 mit dem oberen Teil 903 verbunden. Ein Lenker 936 nutzt die Drehachse des Gelenkes 909 und ist mittels Gelenk 46 mit dem unteren Bereich des Gelenkgliedes 45 verbunden. Der Endbereich 47 des Gelenkgliedes 45 wirkt mit den Führungsflächen 48 in dem unteren Bereich 906 zusammen. Schematisch ist eine Rückstellfeder 933 dargestellt.

Es wird betont, daß aus Gründen der schematischen Darstellung an mehreren Stellen auf die Darstellung von Federmitteln verzichtet worden ist. Es dürfte jedoch klar sein, daß diese an jeder geeigneten Position zwischen miteinander verbundenen Teilen der erfindungsgemäßen Schwenkverbindung angebracht werden können. In entsprechender Weise gilt dies ebenfalls für Dämpfungsvorrichtungen.

Zum Schluß wird darauf hingewiesen, daß funktional übereinstimmende Teile in den einzelnen Ausführungen einheitlich gekennzeichnet sind, wobei den betreffenden Kennzahlen die laufende Nummer der jeweiligen Ausführungsform vorangestellt ist.

## Patentansprüche

1. Schwenkvorrichtung zwischen Teilen eines orthopädischen Hilfsmittels, z.B. Prothesenkniegelenk (1) für Beinamputierte, bestehend aus einer mehrgliedrigen kinematischen Gelenkkette mit mindestens vier Gelenkgliedern (3, 6, 7, 14), in der die jeweils miteinander verbundenen Gelenkglieder eine gemeinsame Drehachse aufweisen, und diese Drehachsen (8, 9, 10, 11) im wesentlichen zueinander parallel verlaufen, **dadurch gekennzeichnet,** daß in der Ausgangsposition des mehrgliedrigen Gelenksystems ein Gelenkglied (14) gegenüber einem mit ihm verbundenen Gelenkglied (6) zwei unterschiedliche Bewegungen ausführen kann, wobei jede dieser beiden Bewegungen eine Rotation, eine Translation oder eine kombinierte Rotation/Translation sein kann, und wobei die Einleitung einer dieser Bewegungen die andere der beiden möglichen Bewegungen zumindest über ihren größten Bewegungsbereich blockiert.

2. Vorrichtung nach Anspruch 1, **dadurch gekennzeichnet,** daß ein Gelenkglied (7) nur mit einem oberen Anschluß-Gelenkglied (3) und mit einem unteren Anschluß-Gelenkglied (6) schwenkbar verbunden ist, wobei die Schwenkbewegung durch wenigstens einen Anschlag (32) größenmäßig begrenzt ist. und ein in Gehrichtung hinter diesem Gelenkglied (7) angeordnetes hinteres Gelenkglied (14) an einem Ende mit dem oberen Anschluß-Gelenkglied (3) schwenkbar verbunden ist und am anderen Ende in der Weise mit dem unteren Anschluß-Gelenkglied (6) verbunden ist, daß das hintere Gelenkglied (14) in der Ausgangsstellung gegenüber dem unteren Anschluß-Gelenkglied (6) sowohl eine Parallelverschiebung als auch eine Schwenkung ausführen kann, wobei ein federndes Element in Verbindung mit wenigstens einem Anschlag dieser Parallelverschiebungs-Bewegung entgegenwirkt, und wobei die Einleitung der Parallelverschiebungs-Bewegung die Schwenkbewegung des hinteren Gelenkgliedes (14) gegenüber dem unteren Anschluß-Gelenkglied (6) zumindest überwiegend blockiert.

3. Vorrichtung nach Anspruch 1, **dadurch gekennzeichnet,** daß ein Gelenkglied (7) nur mit einem oberen Anschluß-Gelenkglied (3) und mit einem unteren Anschluß-Gelenkglied (6) schwenkbar verbunden ist, wobei die Schwenkbewegung durch wenigstens einen Anschlag (32) größenmäßig begrenzt ist, und ein in Gehrichtung hinter diesem Gelenkglied angeordnetes hinteres Gelenkglied (14) an einem Ende mit dem oberen Anschluß-Gelenkglied (3) schwenkbar verbunden ist und am anderen Ende in der Weise mit dem unteren Anschluß-Gelenkglied (6) verbunden ist, daß das hintere Gelenkglied (14) in der Ausgangsstellung gegenüber dem unteren Anschluß-Gelenkglied (6) sowohl eine Parallelverschiebung als auch eine Schwenkung ausführen kann, wobei ein federndes Element in Verbindung mit wenigstens einem Anschlag (20) dieser Schwenkbewegung entgegenwirkt, und wobei die Einleitung der Schwenkbewegung die Parallelverschiebungs-Bewegung des hinteren Gelenkgliedes (14) gegenüber dem unteren Anschluß-Gelenkglied (6) zumindest überwiegend blockiert.

4. Vorrichtung nach Anspruch 1, **dadurch gekennzeichnet,** daß ein Gelenkglied (7) nur mit einem oberen Anschluß-Gelenkglied (3) und mit einem unteren Anschluß-Gelenkglied (606) schwenkbar verbunden ist, wobei die Schwenkung durch wenigstens einen Anschlag (32) größenmäßig begrenzt ist, und ein in Gehrichtung hinter diesem Gelenkglied (7) angeordnetes hinteres Gelenkglied (614) an einem Ende mit dem oberen Anschluß-Gelenkglied (3) schwenkbar verbunden ist und am anderen Ende in der Weise mittels eines Koppelgliedes mit dem unteren Anschluß-Gelenkglied (606) verbunden ist, daß das hintere Gelenkglied (614) in der Ausgangsstellung auf zwei verschiedenen Schwenkkurven gegenüber dem unteren Anschluß-Gelenkglied (606) schwenkbar ist, wobei ein federndes Element in Verbindung mit wenigstens einem Anschlag der einen der beiden Schwenkbewegungen des Koppelgliedes entgegenwirkt, und wobei die Einleitung dieser Schwenkbewegung die andere Schwenkbewegung des hinteren Gelenkgliedes (614) gegenüber dem unteren Anschluß-Gelenkglied (606) zumindest überwiegend blockiert.

5. Vorrichtung nach Anspruch 4, **dadurch gekennzeichnet,** daß das Koppelglied und das federnde Element in struktureller Einheit als Blattfeder (38) ausgeführt sind, die an einer Seite in einem unteren Anschluß-Gelenkglied (606) eingeklemmt und auf der anderen Seite fest oder schwenkbar mit dem nicht mit dem oberen Anschluß-Gelenkglied (3) verbundenen Ende des hinteren Gelenkgliedes (614) verbunden ist.

6. Vorrichtung nach Anspruch 1, **dadurch gekennzeichnet,** daß ein Gelenkglied (707) nur mit einem oberen Anschluß-Gelenkglied (3) und mit einem Koppelglied schwenkbar verbunden ist, wobei die Schwenkung durch wenigstens einen Anschlag (32) größenmäßig begrenzt ist, und ein in Gehrichtung hinter diesem Gelenkglied angeordnetes hinteres Gelenkglied (714) an einem Ende mit dem oberen Anschluß-Gelenkglied (3) schwenkbar verbunden ist und am anderen Ende in der Weise mittels des besagten Koppelgliedes mit dem unteren Anschluß-Gelenkglied (706) verbunden ist, daß das hintere Gelenkglied (714) in der Ausgangsstellung auf zwei verschiedenen Schwenkkurven gegenüber dem unteren Anschluß-Gelenkglied (706) schwenkbar ist, wobei ein federndes Element (39) in Verbindung mit mindestens einem Anschlag der einen der beiden Schwenkbewegungen des Koppelgliedes entgegenwirkt, und wobei die Einleitung dieser Schwenkbewegung die andere Schwenkbewegung des hinteren Gelenkgliedes (714) gegenüber dem unteren Anschluß-Gelenkglied (706) zumindest überwiegend blockiert.

7. Vorrichtung nach Anspruch 1, **dadurch gekennzeichnet,** daß ein Gelenkglied (107) nur mit einem oberen Anschluß-Gelenkglied (103) und mit einem unteren Anschluß-Gelenkglied (106) schwenkbar verbunden ist, wobei die Schwenkung durch wenigstens einen Anschlag (32) größenmäßig begrenzt ist, und ein in Gehrichtung hinter diesem Gelenkglied (107) angeordnetes hinteres Gelenkglied (114) an einem Ende mit dem oberen Anschluß-Gelenkglied (103) schwenkbar verbunden ist und am anderen Ende in der Weise mit dem unteren Anschluß-Gelenkglied (106) verbunden ist, daß das hintere Gelenkglied (114) in der Ausgangsstellung gegenüber dem unteren Anschluß-Gelenkglied (106) sowohl eine Parallelverschiebung als auch eine Schwenkung ausführen kann, wobei ein federndes Element (33) in Verbindung mit wenigstens einem Anschlag dieser Parallelverschiebungs-Bewegung entgegenwirkt, und wobei die Einleitung der Parallelverschiebungs-Bewegung die Schwenkbewegung des hinteren Gelenkgliedes (114) gegenüber dem unteren Anschluß-Gelenkglied (106) zumindest überwiegend durch ein mit dem unteren Anschluß-Gelenkglied verbundenes Koppelglied (132) blockiert.

8. Vorrichtung nach Anspruch 1, **dadurch gekennzeichnet,** daß ein Gelenkglied nur mit einem oberen Anschluß-Gelenkglied und mit einem Koppelglied schwenkbar verbunden ist, wobei die Schwenkung durch wenigstens einen Anschlag größenmäßig begrenzt ist, und ein in Gehrichtung hinter diesem Gelenkglied angeordnetes hinteres Gelenkglied an einem Ende mit dem oberen Anschluß-Gelenkglied schwenkbar verbunden ist und am anderen Ende in der Weise mit dem unteren Anschluß-Gelenkglied verbunden ist, daß das hintere Gelenkglied in der Ausgangsstellung gegenüber dem unteren Anschluß-Gelenkglied sowohl eine Parallelverschiebung als auch eine Schwenkung ausführen kann, wobei ein federndes Element in Verbindung mit wenigstens einem Anschlag einer schwenkenden Parallelverschiebungs-Bewegung des hinteren Gelenkgliedes entgegenwirkt, und wobei die Einleitung der Parallelverschiebungs-Bewegung die Schwenkbewegung des hinteren Gelenkgliedes gegenüber dem unteren Anschluß-Gelenkglied zumindest überwiegend durch eine schwenkende Verbindung des hinteren Gelenkgliedes mit dem besagten Koppelglied blockiert.

9. Vorrichtung nach Anspruch 1, **dadurch gekennzeichnet,** daß mindestens eines der Gelenkglieder (107, 214) mehrfach ausgeführt ist.

10. Vorrichtung nach einem der Ansprüche 2 bis 8, **dadurch gekennzeichnet,** daß mindestens ein zusätzliches federndes Element vorhanden ist, das mit mindestens zwei Gelenkgliedern verbunden ist.

11. Vorrichtung nach Anspruch 1, **dadurch gekennzeichnet,** daß mindestens ein dämpfendes Element vorhanden ist, das mit mindestens zwei Gelenkgliedern verbunden ist.

## Claims

1. Pivoting device between parts of an orthopedic aid, for example a prosthetic knee joint (1) for leg amputees, consisting of a multi-link kinematic linkage, with at least four links (3, 6, 7, 14), in which the links which are joined to one another have a common pivot in each case, and these pivots (8, 9, 10, 11) run substantially parallel to one another,
characterized in that, in the initial position of the multi-link linkage, a link (14) can execute two different motions in relation to a link (6) joined to it, each of these two motions being able to be a rotation, a translation or a combined rotation/ translation, and the initiation of one of these motions inhibiting the other of the two possible motions, at least over its greatest motional range.

2. Device according to Claim 1, characterized in that a link (7) is pivotally joined only to an upper connecting link (3) and to a lower connecting link (6), the pivoting motion being to a large extent limited by at least one stop (32) and a posterior link (14) disposed behind this link (7) in the direction of walking being pivotally joined at one end to the upper connecting link (3) and joined at the other end to the lower connecting link (6) in such a way that, in the initial position, the posterior link (14) can execute both a parallel displacement and a pivoting motion in relation to the lower connecting link (6), a resilient element in combination with at least one stop counteracting this parallel displacement motion, and the initiation of the parallel displacement motion at least predominantly inhibiting the pivoting motion of the posterior link (14) in relation to the lower connecting link (6).

3. Device according to Claim 1, characterized in that a link (7) is pivotally joined only to an upper connecting link (3) and to a lower connecting link (6), the pivoting motion being to a large extent limited by at least one stop (32) and a posterior link (14) disposed behind this link in the direction of walking being pivotally joined at one end to the upper connecting link (3) and joined at the other end to the lower connecting link (6) in such a way that, in the initial position, the posterior link (14) can execute both a parallel displacement and a pivoting motion in relation to the lower connecting link (6), a resilient element in combination with at least one stop (20) counteracting this pivoting motion, and the initiation of the pivoting motion at least predominantly inhibiting the parallel displacement motion of the posterior link (14) in relation to the lower connecting link (6).

4. Device according to Claim 1, characterized in that a link (7) is pivotally joined only to an upper connecting link (3) and to a lower connecting link (606), the pivoting motion being to a large extent limited by at least one stop (32) and a posterior link (614) disposed behind this link (7) in the direction of walking being pivotally joined at one end to the upper connecting link (3) and joined at the other end to the lower connecting link (606), by means of a coupling member, in such a way that, in the initial position, the posterior link (614) can pivot on two different pivoting curves in relation to the lower connecting link (6), a resilient element in combination with at least one stop counteracting one of the two pivoting motions of the coupling member, and the initiation of this pivoting motion at least predominantly inhibiting the other pivoting motion of the posterior link (614) in relation to the lower connecting link (606).

5. Device according to Claim 4, characterized in that the coupling member and the resilient element are formed, in a structural unit, as a leaf spring (38) which on one side is clamped into a lower connecting link (606) and on the other side is rigidly or pivotally joined to the end of the posterior link (614) which is not joined to the upper connecting link (3).

6. Device according to Claim 1, characterized in that a link (707) is pivotally joined only to an upper connecting link (3) and to a coupling member, the pivoting motion being to a large extent limited by at least one stop (32), and a posterior link (714) disposed behind this link in the direction of walking being pivotally joined at one end to the upper connecting link (3) and joined at the other end, by means of the said coupling member, to the lower connecting link (706) in such a way that, in the initial position, the posterior link (714) can pivot on two different pivoting curves in relation to the lower connecting link (706), a resilient element (39) in combination with at least one stop counteracting one of the two pivoting motions of the coupling member, and the initiation of this pivoting motion at least predominantly inhibiting the other pivoting motion of the posterior link (714) in relation to the lower connecting link (706).

7. Device according to Claim 1, characterized in that a link (107) is pivotally joined only to an upper connecting link (103) and to a lower connecting link (106), the pivoting motion being to a large extent limited by at least one stop (32) and a posterior link (114) disposed behind this link (107) in the direction of walking being pivotally joined at one end to the upper connecting link (103) and joined at the other end to the lower connecting link (106) in such a way that, in the initial position, the posterior link (114) can execute both a parallel displacement and a pivoting motion in relation to the lower connecting link (106), a resilient element (33) in combination with at least one stop counteracting this parallel displacement motion, and the initiation of the parallel displacement motion at least predominantly inhibiting the pivoting motion of the posterior link (114) in relation to the lower connecting link (106) through a coupling member (132) joined to the lower connecting link.

8. Device according to Claim 1, characterized in that a link is pivotally joined only to an upper connecting link and to a coupling member, the pivoting motion being to a large extent limited by at least one stop, and a posterior link disposed behind this link in the direction of walking being pivotally joined at one end to the upper connecting link and joined at the other end to the lower connecting link in such a way that, in the initial position, the posterior link can execute both a parallel displacement and a pivoting motion in relation to the lower connecting link, a resilient element in combination with at least one stop counteracting a pivoting parallel displacement motion of the posterior link, and the initiation of the parallel displacement motion at least predominantly inhibiting the pivoting motion of the posterior link in relation to the lower connecting link through the posterior link being pivotally joined to the said coupling link.

9. Device according to Claim 1, characterized in that at least one of the links (107, 214) is of multiple form.

10. Device according to any one of Claims 2 to 8,
characterized in that there is at least one additional resilient element which is joined to at least two links.

11. Device according to Claim 1, characterized in that there is at least one damping element which is joined to at least two links.

## Revendications

1. Dispositif de pivotement entre pièces d'un moyen d'assistance orthopédique, par exemple une articulation de prothèse du genou (1) pour les amputés de la jambe, constitué d'une chaîne cinématique articulée à organes multiples, comprenant au moins quatre organes articulaires (3,6, 7,14) et dans laquelle les organes articulaires reliés l'un avec l'autre présentent à chaque fois un axe de rotation commun, et ces axes de rotation (8, 9, 10,11) s'étendent sensiblement parallèlement entre eux, caractérisé en ce que dans la position de départ du système articulé à organes multiples, un organe articulaire (14) peut exécuter deux mouvements différents par rapport à un organe articulaire (6) qui lui est relié, chacun de ces deux mouvements pouvant être une rotation, une translation ou une rotation/translation combinée, et l'introduction de l'un de ces mouvements bloquant l'autre des deux mouvements possibles au moins sur la plus grande partie de sa plage de mouvement.

2. Dispositif selon la revendication 1, caractérisé en ce qu'un organe articulaire (7) n'est relié qu'avec un organe articulaire de raccordement supérieur (3) et avec un organe articulaire de raccordement inférieur (6) de manière pivotante, le mouvement pivotant étant limité en grandeur par au moins une butée (32), et un organe articulaire postérieur (14), disposé derrière l'organe articulaire (7) précité relativement au sens de la marche, est relié de façon pivotante à une extrémité avec l'organe articulaire de raccordement supérieur (3), et est raccordé à l'autre extrémité avec l'organe articulaire de raccordement inférieur (6) de telle manière que l'organe articulaire postérieur (14) peut effectuer dans la position de départ par rapport à l'organe articulaire de raccordement inférieur (6) aussi bien une translation qu'un pivotement, un élément élastique contrariant ce mouvement de translation en liaison avec au moins une butée, et l'introduction du mouvement de translation bloquant de façon au moins prépondérante le mouvement de pivotement de l'organe articulaire postérieur (14) par rapport à l'organe articulaire de raccordement inférieur (6).

3. Dispositif selon la revendication 1, caractérisé en ce qu'un organe articulaire (7) n'est relié qu'avec un organe articulaire de raccordement supérieur (3) et avec un organe articulaire de raccordement inférieur (6) de manière pivotante, le mouvement de pivotement étant limité en grandeur par au moins une butée (32), et un organe articulaire postérieur (14), disposé derrière l'organe articulaire (7) précité relativement au sens de la marche, est relié de façon pivotante à une extrémité avec l'organe articulaire de raccordement supérieur (3) et est raccordé à l'autre extrémité avec l'organe articulaire de raccordement inférieur (6) de telle manière que l'organe articulaire postérieur (14) peut effectuer dans la position de départ par rapport à l'organe articulaire de raccordement inférieur (6) aussi bien une translation qu'un pivotement, un élément élastique contrariant ce mouvement de pivotement en liaison au moins avec une butée (20), et l'introduction du mouvement de pivotement bloquant de façon au moins prépondérante le mouvement de translation de l'organe articulaire postérieur (14) par rapport à l'organe articulaire de raccordement inférieur (6).

4. Dispositif selon la revendication 1, caractérisé en ce qu'un organe articulaire (7) n'est relié qu'avec un organe articulaire de raccordement supérieur (3) et avec un organe articulaire de raccordement inférieur (606) de manière pivotante, le mouvement de pivotement étant limité en grandeur par au moins une butée (32), et un organe articulaire postérieur (614), disposé derrière l'organe articulaire (7) précité relativement au sens de la marche, est relié de façon pivotante à une extrémité avec l'organe articulaire de raccordement supérieur (3) et est raccordé à l'autre extrémité avec l'organe articulaire de raccordement inférieur (606) au moyen d'un organe de couplage de telle manière que l'organe articulaire postérieur (614) est dans la position de départ pivotant sur deux courbes de pivotement différentes par rapport à l'organe articulaire de raccordement inférieur (606), un élément élastique contrariant l'un des deux mouvements de pivotement de l'organe de couplage en liaison avec au moins une butée, l'introduction de ce mouvement pivotant bloquant de manière au moins prépondérante l'autre mouvement pivotant de l'organe articulaire postérieur (614) par rapport à l'organe articulaire de raccordement inférieur (606).

5. Dispositif selon la revendication 4, caractérisé en ce que l'organe de couplage et l'élément élastique sont réalisés d'une manière structurellement unifiée sous la forme d'une lame de ressort (38) qui est serrée d'un côté dans un organe articulaire de raccordement inférieur (606), et relié de l'autre côté de manière fixe ou pivotante avec l'extrémité de l'organe articulaire postérieur (614) qui n'est pas reliée à l'organe articulaire de raccordement supérieur (3).

6. Dispositif selon la revendication 1, caractérisé en ce qu'un organe articulaire (707) n'est relié qu'avec un organe articulaire de raccordement supérieur (3) et avec un organe de couplage de manière pivotante, le pivotement étant limité en grandeur par au moins une butée (32), et un organe articulaire postérieur (714), disposé derrière l'organe articulaire précité relativement au sens de la marche, est relié de façon pivotante à une extrémité avec l'organe articulaire de raccordement supérieur (3) et est raccordé à l'autre extrémité avec l'organe articulaire de raccordement inférieur (706) au moyen de l'organe de couplage précité, de telle manière que l'organe articulaire postérieur (714) est dans la position de départ pivotant sur deux courbes de pivotement différentes par rapport à l'organe articulaire de raccordement inférieur (706), un élément élastique (39) contrariant l'un des deux mouvements pivotants de l'organe de couplage en liaison avec au moins une butée, l'introduction de ce mouvement pivotant bloquant de manière au moins prépondérante l'autre mouvement pivotant de l'organe articulaire postérieur (714) par rapport à l'organe articulaire de raccordement inférieur (706).

7. Dispositif selon la revendication 1, caractérisé en ce qu'un organe articulaire (107) n'est relié qu'avec un organe articulaire de raccordement supérieur (103) et avec un organe articulaire de raccordement inférieur (106) de manière pivotante, le pivotement étant limité en grandeur par au moins une butée (32), et un organe articulaire postérieur (114), disposé derrière l'organe articulaire (107) précité relativement au sens de la marche, est relié de façon pivotante à une extrémité avec l'organe articulaire de raccordement supérieur (103) et est raccordé à l'autre extrémité avec l'organe articulaire de raccordement inférieur (106) de telle manière que l'organe articulaire postérieur (114) peut effectuer dans la position de départ par rapport à l'organe articulaire de raccordement inférieur (106) aussi bien une translation qu'un pivotement, un élément élastique (33) contrariant ce mouvement de translation en liaison avec au moins une butée, et l'introduction du mouvement de translation bloquant de façon au moins prépondérante le mouvement de pivotement de l'organe articulaire postérieur (114) par rapport à l'organe articulaire de raccordement inférieur (106) au moyen d'un organe de couplage (132) relié avec l'organe articulaire de raccordement inférieur.

8. Dispositif selon la revendication 1, caractérisé en ce qu'un organe articulaire n'est relié qu'avec un organe articulaire de raccordement supérieur et avec un organe de couplage de manière pivotante, le pivotement étant limité en grandeur par au moins une butée, et un organe articulaire postérieur, disposé derrière l'organe articulaire précité relativement au sens de la marche, est relié de façon pivotante à une extrémité avec l'organe articulaire de raccordement supérieur et est raccordé à l'autre extrémité avec l'organe articulaire de raccordement inférieur, de telle manière que l'organe articulaire postérieur peut effectuer dans la position de départ par rapport à l'organe articulaire de raccordement inférieur aussi bien une translation qu'un pivotement, un élément élastique contrariant une translation-pivotement de l'organe articulaire postérieur en liaison avec au moins une butée, l'introduction du mouvement de translation bloquant de manière au moins prépondérante le mouvement de pivotement de l'organe articulaire postérieur par rapport à l'organe articulaire de raccordement inférieur, par une liaison pivotante de l'organe articulaire postérieur avec ledit organe de couplage.

9. Dispositif selon la revendication 1, caractérisé en ce que l'un au moins des organes articulaires (107,214) est réalisé multiple.

10. Dispositif selon l'une des revendications 2 à 8, caractérisé en ce qu'il est prévu au moins un élément élastique supplémentaire qui est relié à au moins deux organes articulaires.

11. Dispositif selon la revendication 1, caractérisé en ce qu'il est prévu au moins un élément amortisseur qui est relié à au moins deux organes articulaires.
